# EUROPÄISCHE PATENTANMELDUNG

(11) **EP 1 477 805 A1**
(43) Veröffentlichungstag der Anmeldung: **17.11.2004**
(21) Anmeldenummer: 03010582.9
(22) Anmeldetag: 12.05.2003
(51) Int. Cl.: G01N 33/50

(54) **Verfahren zur Zellselektion**

(71) Anmelder: ProBioGen AG, 13086 Berlin (DE)
(72) Erfinder: Marx, Uwe, Dr., 13098 Berlin (DE); Demmler, Christian D., 10405 Berlin (DE); Giese, Christoph, Dr., 10439 Berlin (DE)
(74) Vertreter: Helbing, Jörg, Dr. Dipl.-Chem.

(57) **Zusammenfassung**

Die Erfindung betrifft ein Verfahren zur nicht-invasiven, schonenden Selektion einzelner lebender Zellen aus Zellgemischen oder Zellkulturen anhand spezifischer Produktionsleistungen. Hierbei werden die von den einzelnern Zellen produzierte Substanzen, die sich an der Zellmembran anreichern, wie z.B. Reportergenprodukte (GFP) oder aber spezifisch sekretierte Produkte, wie Antikörper über fluoreszenzmikroskopische Nachweisverfahren in ihrer Konzentration bestimmt.

## Beschreibung

Die Erfindung betrifft ein Verfahren zur nicht-invasiven, schonenden Selektion einzelner lebender Zellen aus Zellgemischen oder Zellkulturen anhand spezifischer Produktionsleistungen. Hierbei werden die von den einzelnern Zellen produzierte Substanzen, die sich an der Zellmembran anreichern, wie z.B. Reportergenprodukte (GFP) oder aber spezifisch sekretierte Produkte, wie Antikörper über fluoreszenzmikroskopische Nachweisverfahren in ihrer Konzentration bestimmt.

### Hintergrund der Erfindung

Die Flowcytometrie in Kombination mit einer gezielten Zellsortierung (FACS: Fluorescence Activated Cell Sorting) ist ein bereits seit gut 20 Jahren etabliertes Verfahren. Es erlaubt das schnelle Screening und Sortieren sehr vieler Zellen (Sortierraten bis 2.000 Zellen/sec.). Sie findet aber überwiegend Einsatz im analytischen Bereich (medizinische Diagnostik). Becton Dickinson, Beckman Coulter oder Cytomation bieten entsprechende Gerätetechnik mit Cell-Sorting-Optionen an, die einen zumindest antiseptischen Betrieb ermöglichen kann. Ein steriler oder gar GMP-konformer Prozess kann nicht gewährleistet werden und scheint auch zukünftig nur sehr schwer zu realisieren sein. Zur Gewahrleistung eines hohen Probendurchsatzes wird ein schnell fließendes Fluidiksystem benötigt. Die bei der Passage der Zellen auftretenden Scherkräfte stellen eine massive Beeinträchtigung zellularer Funktionen dar und verringern bei präparativen Anwendungen deutlich die Vitalitat und können zu nachhaltig wirkenden Veränderungen von zellularer Morphologie, Wachstum und Produktivität oder andern spezifischen Funktionen fuhren. Die selektierten Zellen werden außerdem gepoolt; eine sichere Einzelzellablage erscheint für diese Probendurchsatze technisch nicht realisierbar.

Zellen lassen sich in viskosen Medien eingebettet kultivieren (semisolide Matrix). Über einen Sekretionsassay lässt sich mit Hilfe eines fluoreszenz-gekoppelten Detektionssystems ausgeschüttetes Produkt in einer stabilen Wolke um die Zelle stabil präzipitieren. Diese starker als der Hintergrund fluoreszierende Wolke kann zu einer groben Hochproduzentenbewertung genutzt werden. Über eine Bildanalyse als Hochproduzenten identifizierte Zellen können in einem zweiten Schritt über eine mikromanipulator-gesteuerten Glaskapillare manuell oder automatisiert entnommen und weiterkultviert werden (DE-A-10209788). Dieses Verfahren hat jedoch Nachteile bezüglich der Nachweisempfindlichkeit sowie - bedingt durch ein geringes Automatisierungspotential - einen limitierten Probedurchsatz.

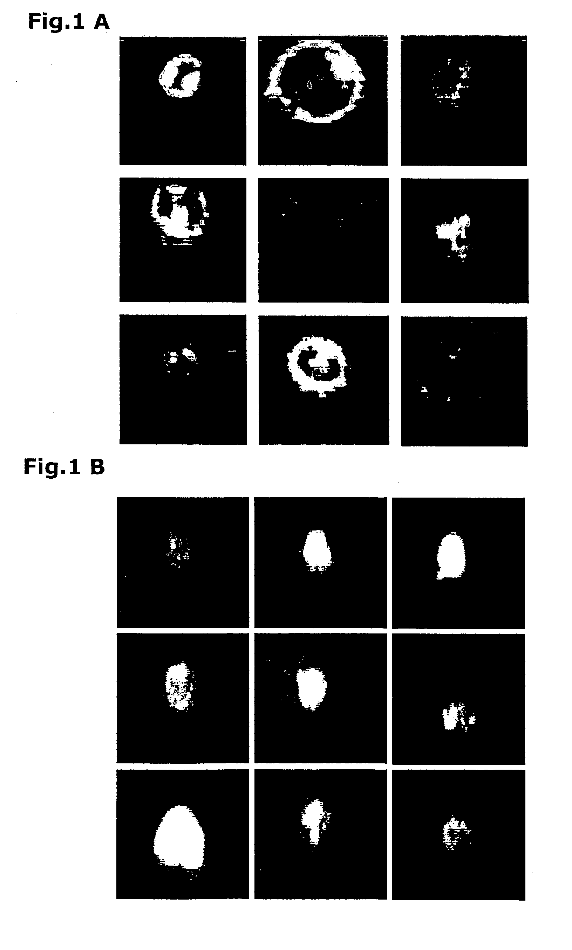

Das US-amerikanische Unternehmen One-Cell-Systems Inc. vermarktet ein besonderes Verfahren zur FACS-gestützten Sortierung von Gel-Mikrotropfen per FACS. Dadurch wird ein zumindest zellschonender Selektionsprozess sichergestellt und mit der großen Sortiergeschwindigkeit der FACS-Technologie kombiniert. Es werden Zellen in Gel-Mikrotropfen von 20-100 µm Durchmesser eingeschlossen. Dort schütten sie ihr Produkt aus, welches an eine speziellen Agarose-Matrix über einen produktspezifischen Fänger-Antikorper gebunden wird. In einem zweiten Schritt wird mit fluoreszenzmarkiertem produktspezifischem Antikörper, das gebundene Produkt nachgewiesen und aufgrund der Lokalisation gleichzeitig die Zahl der Zellen bestimmt (Abb.1b). Das Fluoreszenzsignal kann zum Sortieren der Tropfen mittels FACS-Technologie verwendet werden. Anschließend wir die Matrix wieder aufgelost und die Zellen suspendiert.

Daneben existiert noch die Möglichkeit der Isolation von Zellen mit spezifischer sekretorischer Aktivität über immunomagnetische Separation (Miltenyi, Dynal Biotech, Polysciences). Dieses Verfahren hat jedoch den Nachteil, dass hierdurch keine Quantifizierung der Sekretionsleistung möglich ist und keine Einzelzellspezifität gegeben ist.

Es bestand daher immer noch Bedarf an einem Verfahren, das durch gezielte Auswahl einzelner Zellen und deren nachfolgende Weiterkultivierung, bestimmte Eigenschaften der daraus entstehenden Zellkultur wie z.B. die Produktionsleistung verbessern kann.

### Kurzbeschreibung der Erfindung

Es wurde nun gefunden, dass über die Quantifizierung der an der Zellmembran anhaftenden exprimierten Produkte einer Zelle sowie von fluoreszierenden Produkten in der Zelle oder Zellmembran, z.B. durch fluoreszenzmikroskopisches Verfahren, die Produktkonzentration und somit die Syntheseleistung der jeweiligen Zelle bestimmt werden kann. Die Erfindung betrifft somit ein Verfahren zur Selektion ein spezifisches Produkt produzierender Zellen aus einem Zellgemisch, umfassend die quantitative Bewertung der individuellen Produktionsleistung einer festgehaltenen Zelle anhand des in/an ihr angereicherten Produktes.

Das erfindungsgemäße Verfahren ermöglicht sowohl eine Bestimmung der relativen Syntheseleistung (Ausführungsform A), als auch, aufgrund fluoreszenzspektroskopischer Messung, eine exakte und absolute Konzentrationsbestimmung (Ausführungsform B), aus der über einen standardisierbaren, normierbaren experimentellen Aufbau eine absolute spezifische Produktionsleistung abgeleitet werden kann.

### Kurzbeschreibung der Figuren

Fig.1 zeigt eine Auswahl von Insight-Zellscans des Zellklons MHZ-2 (Fig. 1A) bzw. MHZ-4 (Fig. 1B).
Fig. 2A: Vergleichsmessung der Hüllbildung mittels Flowcytometrie, Vermessung des Fluoreszenzhintergrunds von MHZ-3; FACS-Messung unmarkierter Zellen (MHZ-3)
Fig. 2B Vermessung der produktspezifischen Fluoreszenz (MHZ-3); FACS-Messung produktspezifisch markierter Zellen (MHZ-3),
   jeweils Sekretionsassay mit AntiMaus IgG-fab-RhGr, wobei:
   oben links: Dot-Plot Partikelgranularität vs. Partikelgröße
   oben rechts: Dot-Plot Autofluoreszenz vs. Partikelgröße
   unten: Histogramm der Fluoreszenzintensität (blau: markiert; rot: unmarkiert) FSC-Height: rel. Partikelgröße (Forward-Scatter)
   SSC-Height: rel. Granularität der Partikel (Sideward-Scatter)
   FL1-Height: Fluoreszenz (Kanal 1) bei 530 nm (BP 30) (Anregung: 488/685 nm)

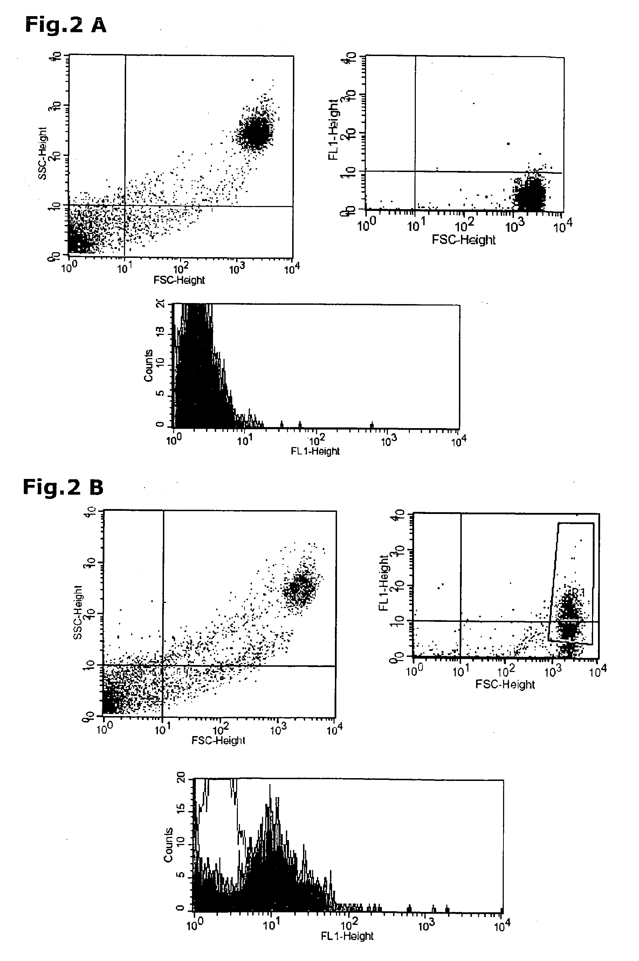

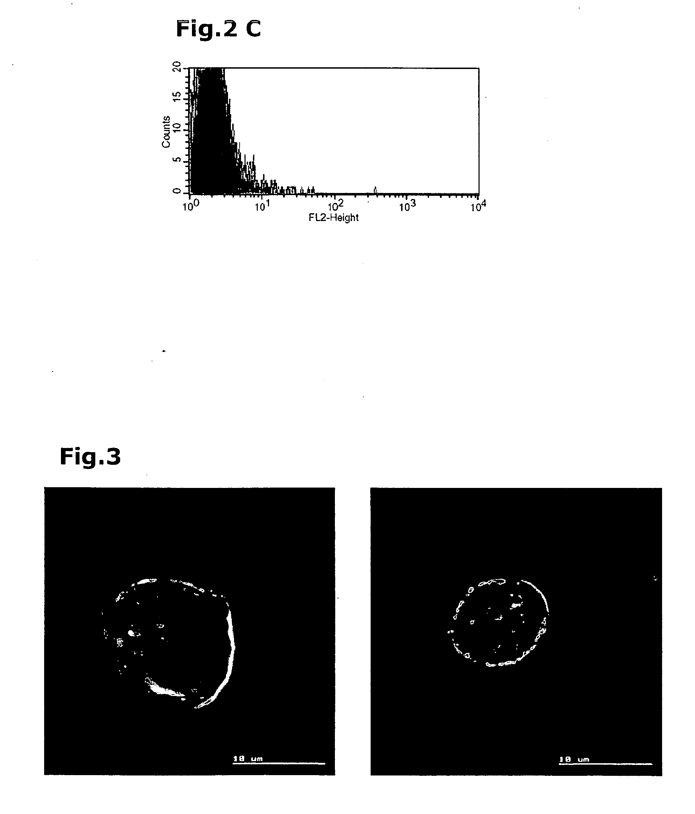

Fig. 2C: Vermessung der Fluoreszenz hervorgerufen durch unspezifische Bindungsereignisse
   FACS-Messung unspezifisch markierter Zellen (MHZ-3) (Sekretionsassay mit Anti Schaf IgG-Texas Red)
   Histogramm der Fluoreszenzintensität (blau: markiert; rot: unmarkiert)
   FL2-Height: Fluoreszenz (Kanal 2) bei 585nm (BP 42) (Anregung: 488 / 685 nm)
Fig. 3 zeigt Paraformaldehyd-fixierte Zellpräparate nach Durchführung des Sekretionsassays mit einem konfokalem Laserscanning-Mikroskop (568 / 590 nm) aufgenommen und mit einem Durchlichtmikroskopbild überlagert.

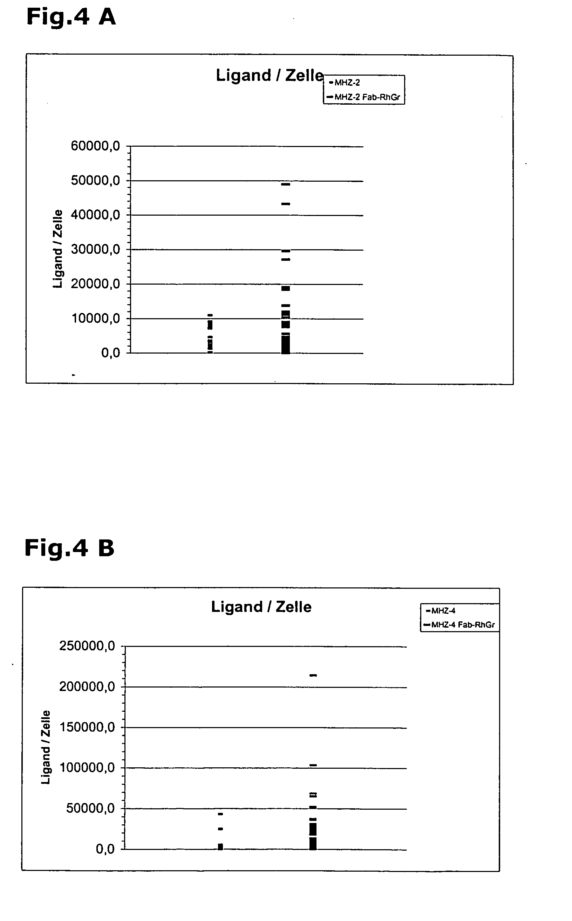

Fig. 4 Vergleichsmessungen von zwei Hybridomaklonen, MHZ-2 (Fig. 4A) und MHZ-4 (Fig. 4B). Die rechten Datenreihen zeigen jeweils die Verteilung von Produktmolekülmengen für 50 vermessene Zellen, die linken Datenreihen Signale unmarkierter Zellen (Hintergrundsignale).

### Detaillierte Beschreibung der Erfindung

Bei dem erfindungsgemäßen Verfahren kann das Produkt in der Zelle, in der Zellmembran oder an der Zellmembran lokalisiert (sezerniert) sein. Diese unterschiedliche Lokalisierung des Produkts ist in der nachfolgenden Skizze näher erläutert:

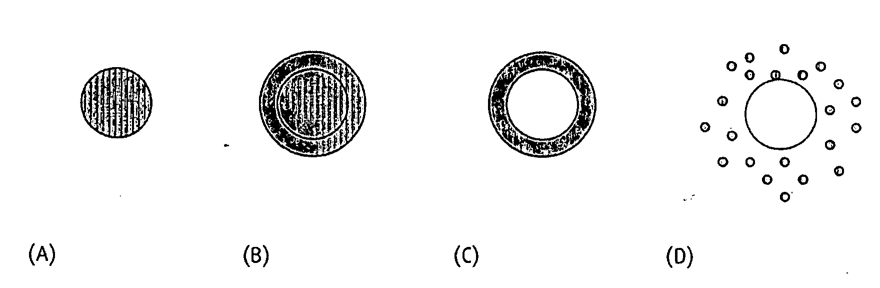

### Fluoreszenzerscheinungen mit unterschiedlicher zellulärer Lokalisation (nur intrazellulär (A), intrazellulär und membranständig (B), nur membranständig (C) sowie perizellulär (D))

Die Bestimmung der Produktionsleistung kann dabei lichtmikroskopisch oder durch fluoreszenzspektroskopische Verfahren erfolgen. Ein wichtiger Aspekt des erfindungsgemäßen Verfahrens ist, dass die Bestimmung unter Fixierung der Zelle erfolgt. Anhand der erfassten Produktionsleistung lässt sich dann unmittelbar eine Klassifizierung über definierte Schwellenwerte in zu selektierende bzw. nicht zu selektierende Zellen durchführen.

Durch das erfindungsgemäße Verfahren kann das Produkt, ausgewählt aus Proteinen, Glycosiden und Derivaten und Kombinationsprodukten aus diesen Substanzklassen usw. bestimmt werden. Die Produkte sind vorzugsweise ausgewählt aus Proteinen und Peptiden wie Antikörpern, Antikörperfragmenten, Cytokinen, usw., sowie glykosylierte und/oder mit nachweisbaren Markern oder mit fluoreszenzierenden Domänen versehene Derivate von Proteinen und Peptiden. In dem erfindungsgemäßen Verfahren kann das Produkt von einer Primärzelle oder als Folge einer Manipulation an der Zelle von dieser exprimiert werden, d. h. es können dabei Primärzellen (d. h. unmanipulierte Zellen) oder Transformanten eingesetzt werden. In dem erfindungsgemäßen Verfahren kann das Produkte direkt nachweisbar sein und insbesondere eine fluoreszenzmikroskopisch oder -spektroskopisch nachweisbare Domäne aufweisen, oder indirekt durch Komplexierung mit einer Detektorverbindung, insbesondere fluoreszenzmarkierte Antikörper nachweisbar sein.

Das erfindungsgemäße Verfahren kann weiterhin einen Selektionsschritt umfassen, bei dem vorzugsweise das Zellgemisch durch ein Mikrofluidiksystem geleitet wird. Hierbei ist bevorzugt, dass die einzelnen Zellen aus fließender Bewegung im Mikrofluidiksystem als solche identifiziert, gezielt angehalten und unter kontinuierlicher Beobachtung ihrer Sekretionsleistung erfasst werden. Das Festhalten (Fixieren) der Zelle erfolgt besonders bevorzugt berührungsfrei durch einen elektrostatischen Feldkäfig (z. B. durch neg. Dielektrophorese).

In einer weiteren bevorzugten Ausführungsform des erfindungsgemäßen Verfahrens kann das Produkt selbst fluoresziert und seine Konzentration aus dem gemessenen Fluoreszenzsignal abgeleitet werden. Alternativ hierzu kann das Produkt über eine Komplexbildung durch Rezeptor-Ligand-Wechselwirkung quantifiziert werden, welche fluoreszenzspektroskopisch erfassbar ist und eine Bestimmung der Produktkonzentration ermöglicht. Hierbei erfolgt die Komplexbildung vorzugsweise durch Antigen-Antikörper-Wechselwirkung, welche fluoreszenzspektroskopisch erfassbar ist und eine Bestimmung der Produktkonzentration ermöglicht.

Die bei dem erfindungsgemäßen Verfahren selektionierten Zellen können gezielt durch Ableitung in einen abzweigenden Kanal vom Probenstrom abgetrennt und in ein geeignetes Kultursystem abgelegt werden. Dieses Ableiten in einen abzweigenden Kanal erfolgt vorzugsweise durch Öffnen elektrostatischer Tore/Schleusen. Es ist ein nennenswertes Merkmal des erfindungsgemäßen Verfahrens, dass die Ablage der selektierten Zellen steril erfolgen kann.

Das Kultivieren der Zellen erfolgt vorzugsweise in einem Kultursystem, das Kultursystem für die Kultur einzelner oder weniger Zellen optimiert ist (für Säugerzellen, z. B. humane, murie und bovine Zellen (primär, transformiert und/oder immortalisiert)) und durch Supplementierung, gezielte Konditionierung oder spezielle Formulierung optimiert ist. Das Kultursystem kann dabei miniaturisiert sein, was bedeutet, dass das Kulturvolumen reduziert ist. Weiterhin kann das Kulturmedium durch Verwendung einer biogenen oder artifiziellen extrazellulären Matrix unterstützt werden.

Das erfindungsgemäße Verrfahren ist insbesondere zur Identifizierung von Zellen mit hoher Produktionsleistung oder Sekretionsleistung und/oder Optimierung von Zellkultur-basierenden Produktionsprozessen geeignet.

Die Erfindung wird anhand der folgenden Beispiele näher erläutert, die jedoch den Schutzumfang des Patents nicht einschränken sollen.

### Beispiele

### Materialien und Methoden

Assay: Die Vorbereitung der Zellen für das Cytocon-System in Kombination mit einer FCS+plus-Messung efolgte durch die folgenden Schritte:
Zellüberstand abnehmen und Zellzahl (Vitalität) mit dem Coulter Z2 bestimmen; 1 x 10⁶ vitale Zellen in ein 15 ml Falcon + 10 ml PBS geben und bei 100 g 10 min abzentrifugieren;
Überstand bis auf minimales Restvolumen verwerfen und Pellet in 5 µl RhGr-Fab resuspendieren und 30 min bei 37 °C inkubieren;
10 ml PBS zugeben, resuspendieren und bei 100 g; 10 min abzentrifugieren;
Überstand abnehmen, Pellet in 2 ml Cytocon-Puffer II (1:4) aufnehmen und Injektionsspritze (100 µl) befüllen

Verwendete Zelllinien: Für die Selektionsexperimente im Rahmen der Machbarkeitstudie haben wir uns für zwei Zelllinien entschieden, die in Produktionsprozessen der Anmelderin von Bedeutung und dadurch umfassend charakterisiert sind (MHZ-2; MHZ-4). Für flowcytometrische Messungen wurde die Zelllinie MHZ-3 eingesetzt. Es handelt sich in allen Fallen um monoklonale Maus-Maus-Hybridomazelllinien, die einen IgG-Antikorper produzieren und ausschütten. Die Produktionsleistungen liegen in klassischer Erhaltungskultur (10³-10⁶ Zellen/ml) für MHZ-2
bei 5-50 ng pro 1.000 Zellen und Tag. Dies entspricht einer spezifischen Produktionsrate an IgG
von 60-600 Molekülen pro Zelle und s.
In Cell-Line-Kultur lassen sich für die verwendeten Zellklone Produktivitäten abschätzen, die sich
auf 1-4 10⁷ Zellen/ml Kulturvolumen beziehen lassen. Die Produktkonzentrationen wurden über
Anti-Maus Fcy-spezifischen ELISA bestimmt.

| | |
|---|---|
| MHZ-2 | 40-100 µg/Tag |
| MHZ-4 | 400-800 µg/Tag |

Die Produktivität von MHZ-4 ist bis um den Faktor 10 hoher als die von MHZ-2.

Detektionssystem: Im Rahmen der Machbarkeitstudie wurden in umfassenden Messreihen verschiedene Detektionssysteme für die Assay-Entwicklung getestet. Dabei handelte es sich um kommerziell erhältliche Antikörperkonjugate für die Immunfluoreszenzmikroskopie (IgG-Texas Red), bakterielles rekombinantes Protein A und Protein G sowie ein Spaltprodukt eines IgG-Antikorpers, ein f(ab)-Praparat.

### Beispiel 1: Quantifizierung der Sekretionsleistung durch Insight-Scans anhand der Präzipitathülle

Hierbei erfolgt eine Vermessung der Präzipitathülle, die sich über eine definierte Inkubationszeit um eine Zelle bildet. Detektionsmoleküle bilden mit Produktmolekülen ein die Zelle umgebene Prazipitathülle, die in Ihrer Mächtigkeit der ausgeschütteten Produktmenge entspricht. Die Vermessung der zellgebundenen Fluoreszenzerscheinung lasst eine Bestimmung der zellspezifischen Produktionsrate zu. Die Konzentrationsbestimmung an Produkt kann messtechnisch entweder fluoreszenzspektroskopisch mittels Insight-Technik, aber auch fluoreszenzmikroskopisch über ein Imaging-Verfahren erfolgen. Die Messsignale der fluoreszierenden Prazipitathülle sind wesentlich höher und lassen sich mit den getesteten Detektionssystemen problemlos erfassen. Das Imaging-Verfahren bedeutet eine wesentliche Beschleunigung des Selektionsprozesses sowie ein einfacheres und sicheres Bewertungsverfahren. Es erlaubt aber keine absolute Konzentrationsbestimmung, sondern lediglich eine relative Klassifizierung von Zellen aufgrund einer Bild- bzw. Objekthelligkeitsbewertung.

Die nach dem standardisierten Assayprotokoll gefärbten Zellen (2 Hybridoma-Zellkone) zeigen eine produktspezifische Fluoreszenz in Form einer Hülle. Die Stärke der Hüllbildung ist proportional zur tatsächlichen Sekretionsleistung (Messung sekretierten Produkts im Kultüberstand verschiedener Klone). Mit fortschreitender Inkubationszeit nimmt die Hüllenbildung zu. Sie bleibt nach Beendigung der Inkubationszeit durch den Waschvorgang über 80 min auf konstantem Niveau erhalten. Die Zellen zeigen nach Sekretion-Assay eine deutliche und individuell unterscheidbare Fluoreszenzerscheinung. Diese ist überwiegend extrazellulär und membranständig lokalisierbar. Sie kann aber auch flächenhaft oder granulär im Cytoplasma, wie bei MHZ-4, auftreten. Die spezifische Hüllbildung und deren differenzielle Fluoreszenz wurde sowohl in Insight-Scans (Fig.1A und B), in flowcytometrischer Untersuchung (FACS; siehe Fig. 2A-C) und im konfoklalen Laserscanning-Mikroskop (LSM; siehe Fig.3) nachgewiesen. Die Zellen zeigen eine Eigenfluoreszenz. Diese kann von der spezifischen Fluoreszenz durch Setzen eines Schwellenwertes unterschieden werden.

Im Feldkäfig werden Artefakte beobachtet, die granulär aussehen. Dies liegt an der Natur der negativen Dielektrophorese im Feldkäfig. Es werden alle möglichen Partikel im Käfigbereich angereichert (z. B. auch gefärbte Zelltrümmer und Sondenaggregate) und an die Oberfläche der Zelle geheftet. Dies interferiert nicht mit dem Messverfahren und haben keinen Einfluss auf die Vitalität der Zellen.

Datenanalyse: Für MHZ-2 und MHZ-4 ergibt sich aufgrund der Eigenfluoreszenz der Zellen ein Hintergrundrauschen von bis zu 10.000, bzw. 50.000 Liganden/Zelle. Sekretionsspezifische Signale reichen von 10.000 bis 50.000 bzw. 50.000 bis 200.000 Liganden/Zelle (siehe Fig. 4A und 4B) Eine Hochproduzentenselektion erfolgt für MHZ-2 bei Werten über 30.000 und für MHZ-4 bei Werten über 100.000 Liganden/Zelle.

Aus ELISA-Vergleichsmessungen von Überständen aus der etablierten Kultur der beiden ausgewählten Klone (Anti-Maus Fcy-spezifisch) folgende Produktivitäten bestimmt:

| | |
|---|---|
| MHZ-2 | 40-100 µg/Tag |
| MHZ-4 | 400-800 µg/Tag |

Demgemäß ist die Produktivität von MHZ-4 annähernd um den Faktor 10 höher als die von MHZ-2. Dieser Befund wurde durch die hier vermessene kleine Zahl an Zellen bestätigt.

Ablage selektierter Zellen und Nachweis des Auswachsens von Klonen: In Kulturexperimenten mit wie vorstehend beschrieben abgelegten Zellen konnte gezeigt werden, dass diese Zellen vergleichbares Anwachsverhalten zeigen wie mit konventionellen Pipettierverfahren abgelegte Zellen (Kulturzeit 300 h; in Standardmedium). Weiterhin konnte gezeigt werden, dass das Selektionsmerkmal über eine Kulturdauer von 71 d (entspricht 44 bzw. 47 Zellteilungszyklen) stabil bleibt.

## Patentansprüche

1. Verfahren zur Selektion ein spezifisches Produkt produzierender Zellen aus einem Zellgemisch, umfassend die quantitative Bewertung der individuellen Produktionsleistung einer festgehaltenen Zelle anhand des in/an ihr angereicherten Produktes.

2. Verfahren nach Anspruch 1, wobei
(i) das Produkt in der Zelle, in der Zellmembran oder an der Zellmembran lokalisiert (sezerniert) sein kann; und/oder
(ii) die Bestimmung der Produktionsleistung lichtmikroskopisch oder durch fluoreszenzspektroskopische Verfahren erfolgt; und/oder
(iii) die Bestimmung unter Fixierung der Zelle erfolgt; und/oder
(iv) anhand der erfassten Produktionsleistung unmittelbar eine Klassifizierung-über definierte Schwellenwerte in zu selektierende bzw. nicht zu selektierende Zellen erfolgt.

3. Verfahren nach Anspruch 1 oder 2, wobei
(i) das Produkt ausgewählt ist aus Proteinen, Glycosiden und Derivaten und Kombinationsprodukten aus diesen Substanzklassen und vorzugsweise ausgewählt ist aus Proteinen und Peptiden wie Antikörpern, Antikörperfragmenten, Cytokinen, usw., sowie glykosylierte und/oder mit nachweisbaren Markern oder mit fluoreszenzierenden Domänen versehene Derivate von Proteinen und Peptiden; und/oder
(ii) das Produkt von einer Primärzelle oder als Folge einer Manipulation an der Zelle von dieser exprimiert wird; und/oder
(iii) das Produkte direkt nachweisbar ist und insbesondere eine fluoreszenzmikroskopisch oder -spektroskopisch nachweisbare Domäne aufweist, oder indirekt durch Komplexierung mit einer Detektorverbindung, insbesondere fluoreszenzmarkierte Antikörper nachweisbar ist.

4. Verfahren nach einem oder mehreren der Ansprüche 1 bis 3, wobei das Verfahren weiterhin einen Selektionsschritt umfasst, bei dem vorzugsweise
(i) das Zellgemisch durch ein Mikrofluidiksystem geleitet wird; und/oder
(ii) die einzelnen Zellen aus fließender Bewegung im Mikrofluidiksystem als solche identifiziert, gezielt angehalten und unter kontinuierlicher Beobachtung ihrer Sekretionsleistung erfasst werden; und/oder
(iii) das Festhalten (Fixieren) der Zelle berührungsfrei durch einen elektrostatischen Feldkäfig erfolgt (neg. Dielektrophorese).

5. Verfahren nach einem oder mehreren der Ansprüche 1 bis 4, wobei
(i) das Produkt selbst fluoresziert und seine Konzentration aus dem gemessenen Fluoreszenzsignal abgeleitet werden kann; oder
(ii) eine Komplexbildung durch Rezeptor-Ligand-Wechselwirkung erfolgt, welche fluoreszenzspektroskopisch erfassbar ist und eine Bestimmung der Produktkonzentration ermöglich, wobei die Komplexbildung vorzugsweise durch Antigen-Antikörper-Wechselwirkung erfolgt, welche fluoreszenzspektroskopisch erfassbar ist und eine Bestimmung der Produktkonzentration ermöglicht.

6. Verfahren nach einem oder mehreren der Ansprüche 1 bis 5, wobei
(i) die zu selektierende Zellen gezielt durch Ableitung in einen abzweigenden Kanal vom Probenstrom abgetrennt und in ein geeignetes Kultursystem abgelegt werden; und/oder
(ii) dieses Ableiten in einen abzweigenden Kanal vorzugsweise durch Öffnen elektrostatischer Tore/Schleusen erfolgt; und/oder
(iii) die Ablage der selektierten Zellen steril erfolgt

7. Verfahren nach Anspruch 6, wobei
(i) das Kultursystem für die Kultur einzelner oder weniger Zellen optimiert ist; und/oder
(ii) das Kultursystem miniaturisiert und das Kulturvolumen reduziert ist; und/oder
(iii) das Kulturmedium für die Kultur einzelner oder weniger Zellen durch Supplementierung, gezielte Konditionierung oder spezielle Formulierung optimiert ist; und/oder
(iv) das Kulturmedium durch Verwendung einer biogenen oder artifiziellen extrazellulären Matrix unterstützt ist.

8. Verfahren nach einem oder mehreren der Ansprüche 1 bis 7, das zur Identifizierung von Zellen mit hoher Produktionsleistung oder Sekretionsleistung und/oder Optimierung von Zellkultur-basierenden Produktionsprozessen geeignet ist.
